# EUROPÄISCHE PATENTANMELDUNG

(11) **EP 4 497 814 A1**
(43) Veröffentlichungstag der Anmeldung: **29.01.2025**
(21) Anmeldenummer: 23188450.3
(22) Anmeldetag: 28.07.2023
(51) Int. Cl.: C12M 1/24, C12M 1/12, C12M 3/04

(54) **VORRICHTUNG ZUR VERMEHRUNG VON DIFFERENZIERTEN UND UNDIFFERENZIERTEN ZELLEN UND VERWENDUNG DER VORRICHTUNG FÜR DIE HERSTELLUNG VON KULTIVIERTEN LEBENSMITTELN**

(71) Anmelder: Green Elephant Biotech GmbH, 35394 Gießen (DE)
(72) Erfinder: Eichmann, Joel, 35392 Gießen (DE); Käßer, Lukas, 35460 Staufenberg (DE)
(74) Vertreter: Metten, Karl-Heinz

(57) **Zusammenfassung**

Die vorliegende Erfindung betrifft eine Vorrichtung zur Vermehrung von Zellen, umfassend ein Gehäuse mit einer sich von einem ersten Gehäuseende bis zu einem zweiten Gehäuseende erstreckenden longitudinalen Orientierung, eine spiralförmig um eine entlang oder parallel zu der longitudinalen Orientierung ausgerichtete Wickelachse aufgewickelt in dem Gehäuse vorliegende Kunststofffolie mit einem ersten und einem zweiten Folienende sowie mit einer Innen- und einer gegenüberliegenden Außenseite, wobei auf der Innenseite oder auf der Außenseite oder auf der Innen- und der Außenseite der Kunststofffolie eine Vielzahl an Abstandshaltern vorliegt, eingerichtet und ausgelegt, die Innenseite der gewickelten Kunststofffolie abschnittsweise oder im Wesentlichen vollständig im aufgewickelten Zustand beanstandet zu halten von der Außenseite, sodass entlang der longitudinalen Orientierung, insbesondere vom ersten Gehäuseende bis zum zweiten Gehäuseende ein flüssiges Medium transferierbar ist. Ferner betrifft die Erfindung die Verwendung der erfindungsgemäßen Vorrichtung für die Herstellung von kultivierten Lebensmitteln oder für die Gewinnung von vermehrten differenzierten oder undifferenzierten Zellen.

## Beschreibung

Die vorliegende Erfindung betrifft eine Vorrichtung zur Vermehrung von differenzierten und undifferenzierten Zellen. Ferner betrifft die Erfindung die Verwendung der erfindungsgemäßen Vorrichtung zur Vermehrung von differenzierten und undifferenzierten Zellen. Außerdem betrifft die Erfindung die Verwendung der erfindungsgemäßen Vorrichtung für die Gewinnung von vermehrten differenzierten wie auch undifferenzierten Zellen. Schließlich betrifft die Erfindung die Verwendung der erfindungsgemäßen Vorrichtung für die Herstellung von kultivierten Lebensmitteln wie kultiviertem Fleisch.

Nahrungsmittel auf Fleischbasis wie auch die Nutzung von Fischen und Meeresfrüchten als Nahrungsmittel werden von vielen Verbrauchern aus unterschiedlichsten Gründen mehr und mehr hinterfragt. In diese Überlegungen spielen Aspekte der Massentierhaltung ebenso hinein wie ethische Bedenken grundsätzlicher Art und Erwägungen der Nachhaltigkeit. So ist der Beitrag der industriellen Fleischproduktion zum *Global Warming* nicht gering zu schätzen, sind doch Rinderherden weltweit für einen großen Teil der gesamten Methanemissionen verantwortlich. Unbestritten ist zur Erzeugung einer Kilokalorie aus Fleisch ein Mehrfaches dieses Nährwerts in Form von Futterpflanzen nötig. Dass der Bedarf an fleischlosen Nahrungsmitteln bei einer stetig wachsenden Weltbevölkerung angesichts einer im Wachstum begriffenen Nachfrage nach derartigen Nahrungsmitteln bedient werden kann, wird als kritisch erachtet. Seit einiger Zeit gibt es daher Versuche, auf künstlichem Wege zu Fleischprodukten, sogenanntem kultivierten Fleisch bzw. in-vitro-Fleisch, zu gelangen.

Aus der DE 42 00 446 A1 geht ein Bioreaktor, der mit einer Zellträgeranordnung ausgestattet ist, hervor, mit der in optimaler Weise Biomaterial wie Zellen produziert werden können. Hierfür hat die Zellträgeranordnung wenigstens doppelwandig ausgebildet zu sein und aus wenigstens zwei Zuschnitten aus einem Flachmaterial zu bestehen, wobei die Zuschnitte zwischen sich wenigstens einen Spalt bzw. einen spaltartigen Bereich zu bilden haben.

Die DE 22 36 240 A1 behandelt eine Vorrichtung zur Züchtung von Gewebezellen und Mikroorganismen, bei der die in einem Behälter angeordnete Fläche in einer Spiralwindung auf einer im Wesentlichen horizontalen, drehbaren Welle so aufgewunden ist, dass zwischen den Windungen ein Abstand gehalten wird. Hierbei hat die rotierende Fläche an ihren Stirnseiten durch je eine Scheibe gegen die Behälter abgeschlossen zu sein, und die Scheiben haben an dem der innersten Windung benachbarten Teil Öffnungen aufzuweisen. Auf diese Weise soll auf einfache Weise die Züchtung von Gewebezellen auf großflächiger Oberfläche gelingen, wobei die erhaltenen Gewebezellen leicht geerntet werden können.

Die DE 195 46 542 C1 thematisiert ein Zellkultivierungsgefäß mit einer Zellkulturkammer, in der ein flächenhaft ausgebildeter Träger für die Zellkultur aus einem flexiblen, als Band aus einer Membran oder einem Gewebe gebildeten Material zu einer Spirale aufgewickelt und an einem Halteelement aus einem formstabilen Material befestigt ist. Die Zellkulturkammer hat einerseits von einer für die Aufnahme des Versorgungsmediums vorgesehenen Versorgungskammer durch eine semipermeable Membran und andererseits von der Umgebungsatmosphäre durch eine für Flüssigkeiten undurchlässige, für Gase jedoch durchlässige Gasaustauschmembran getrennt zu sein. Dieses Zellkultivierungsgefäß soll einfach handhabbar und preisgünstig herstellbar sein und ein Zellkulturverfahren ermöglichen, mit dem hohe Zelldichten erreichbar sind.

Die DE 10 2010 005 415 A1 betrifft eine Vorrichtung, mit der adhärent oder in Suspension wachsende Stammzellen und primäre Zellen aus humanen oder tierischen Quellen in einem Drehbett-Bioreaktor dynamisch und kontinuierlich im Perfusionsmodus expandiert werden können. Diese Vorrichtung zur Züchtung, Vermehrung und/ oder Differenzierung von in Kulturmedien suspendierten primären Zellen oder Stammzellen besteht aus einem Reaktorgefäß mit einem demontierbaren Verschlussdeckel, regelbaren Anschlüssen für die Zu- und Abflüsse von Medien, Anschlüssen für die Zu- und Abführung von Overlay-Atmosphäre und einer im Reaktorgefäß axial angeordneten, über einen Magnetantrieb berührungslos angetriebenen rotierenden Welle. Auf der Welle sind ein oder mehrere aus Zellträgern bestehende Drehbetten angeordnet, die mittels des berührungslosen Magnetantriebes alternierend durch ein Kulturmedium und durch die sich im Kopfraum des Reaktorgefäßes befindliche Overlay-Atmosphäre bewegt werden. Die rotierbare Welle hat im Reaktorgefäß auf der einen Seite lösbar und auf der anderen Seite des Reaktorgefäßes in einem vom Reaktorgefäß demontierbaren Deckel gelagert zu sein. Das Drehbett hat aus einem Paket von übereinander, mit einem definierten Abstand zueinander, parallel zur Welle des Drehbettes angeordneten Zellträgern in Form rechteckiger Scheiben unterschiedlicher, jeweils mit einer an den Durchmesser des Reaktorgefäßes angepassten Breite und einer Dicke von 0,2 mm bis 2 mm und runden Stirnscheiben mit entsprechend dimensionierten Rillen zur Fixierung der rechteckigen Scheiben zu bestehen.

In der EP 3 071040 B1 wird ein in-vitro-Verfahren zum Herstellen eines kultivierten Fleischprodukts beschrieben, umfassend i) das Modifizieren einer sich selbst erneuernden Zelllinie von Nutztier-, Geflügel-, Wild- oder Wassertierarten mit einem induzierbaren myogenen Transkriptionsfaktor, um eine mit einem myogenen Transkriptionsfaktor modifizierte Zelllinie zu erzeugen, wobei die sich selbst erneuernde Zelllinie eine pluripotente embryonale Stammzelllinie oder eine induzierte pluripotente Stammzelllinie ist, ii) das Halten der modifizierten Zelllinie in einem Selbsterneuerungsprozess und anschließendem Induzieren einer myogenen Differenzierung der modifizierten Zelllinie durch exogene Regulation des induzierbaren myogenen Transkriptionsfaktors, wobei das Induzieren der myogenen Differenzierung ferner das Inberührungbringen der modifizierten Zelllinie mit einem DNA-Methylierungshemmer umfasst und wobei die differenzierte modifizierte Zelllinie Myozyten und mehrkernige Myotuben ausbildet, die jeweils Myonuklei umfassen, und iii) das Kultivieren der Myozyten und der mehrkernigen Myotuben, um ein kultiviertes Fleischprodukt zu erzeugen. Mit diesem Verfahren soll die skalierbare in vitro-Züchtung von Fleisch aus einer sich selbst erneuernden Quelle für diätetische Ernährung gelingen.

Aus der WO 2019/211189 A1 geht eine Vorrichtung mit einem langgestreckten Körper zur Herstellung von Gewebe aus Zellen hervor. Der langgestreckte Körper hat mindestens eine Umfangsnut aufzuweisen und erstreckt sich im Wesentlichen bündig mittig durch einen sich in einem geschlossenen Weg erstreckenden Trog, wobei sich mindestens eine der Umfangsnuten in eine Innenkante des Trogs öffnet. Mit dieser Vorrichtung soll künstliches Muskelfleisch mit einem automatisierten und skalierbaren Verfahren zugänglich sein.

In der CA 2 566 841 C wird abgestellt auf einen Bioreaktor, umfassend eine Membranträgerstruktur und eine hochporöse Membran, die auf der Membranträgerstruktur aufliegt. Die Membran weist eine Nährstoffseite und eine sogenannte Gasseite auf. Ferner hat die Membran eine immobilisierte Bioschicht zu enthalten. Die Membran hat derart eingerichtet und ausgelegt zu sein, dass sie die Diffusion einer Nährlösung von der Nährstoffseite zu der immobilisierten Bioschicht ohne äußeren Druck ermöglicht. Schließlich haben die Membrane in Paaren auf der Membranträgerstruktur angeordnet zu sein und einen Innenbereich zu definieren. Der Bioreaktor der CA2 566 841 C soll kostengünstig und langlebig sein und höhere Biokonversionsraten als herkömmliche Systeme ermöglichen.

Aus der CA 3 086 283 A1 ist ein Bioreaktor zum Kultivieren von Zellen bekannt, umfassend ein Basisteil mit einer ersten Kammer enthaltend einen Rührer und eine erste zentrale Säule, die abnehmbar an dem Basisteil angebracht ist, wobei die erste zentrale Säule mindestens einen Teil einer zweiten, äußeren Kammer zum Züchten von Zellen und eine dritte innere Kammer zum Zurückführen des Fluidstroms von der zweiten äußeren Kammer zu der ersten Kammer bildet. Auf diese Weise soll ein modular aufgebauter Bioreaktor bereitgestellt werden, mit dem ein oder mehrere strukturierte Festbetten verwendet werden können, um den Herstellungsprozess zu vereinfachen und gleichzeitig hervorragende Zellkultivierungsergebnisse in Sachen Homogenität und Wiederholbarkeit zu erzielen.

Die EP 3 068 866 B1 offenbart ein System zur Zellexpansion, enthaltend einen Bioreaktor, einen Antrieb zum Drehen des Bioreaktors, eine Fluidzirkulationseinheit, die fluidisch mit dem Bioreaktor in Verbindung steht, eine Pumpe zum Zirkulieren von Fluid durch die Fluidzirkulationseinheit und den Bioreaktor, einen Prozessor und einen Speicher, der die prozessorgesteuerten Anweisungen speichert. Mit diesem System sollen sich Zellen in einem Bioreaktor, der mit einem Zellexpansionssystem verbunden ist, unproblematisch laden und verteilen lassen.

Die WO 2020/ 163329 A1 betrifft eine Zellkulturmatrix, enthaltend ein Substrat, umfassend eine erste Seite, eine gegenüberliegende zweite Seite und eine Vielzahl von Öffnungen, die in dem Substrat ausgebildet sind und die durch das Substrat hindurchgehen. Die Vielzahl von Öffnungen hat dabei so konfiguriert zu sein, dass sie den Durchfluss eines Zellkulturmediums, von Zellen oder von Zellprodukten zulässt. Mit der technischen Lehre der WO 2020/ 163329 A1 sollen Zellkulturmatrizen für die Kultivierung von Zellen mit hoher Dichte, gleichmäßiger Zellverteilung und erhöhten Ernteerträgen ermöglicht werden.

Die Verfahren zur Herstellung und Gewinnung von kultiviertem Fleisch sind stets sehr aufwendig und in der Regel nicht für eine Produktion im industriellen Maßstab geeignet. Der vorliegenden Erfindung hat daher die Aufgabe zugrunde gelegen, Verfahren bzw. Vorrichtungen für die Gewinnung von kultiviertem Fleisch verfügbar zu machen, die nicht mehr mit den Nachteilen des Stands der Technik behaftet sind und die insbesondere unkompliziert eingesetzt werden können und zuverlässig einen hohen Ertrag gewährleisten. Insbesondere lag der Erfindung die Aufgabe zugrunde, Vorrichtungen zur Gewinnung von kultiviertem Fleisch verfügbar zu machen, die ein nachhaltiges Wirtschaften ermöglichen.

Demgemäß wurde eine Vorrichtung zur Vermehrung von Zellen wie differenzierten und undifferenzierten Zellen, insbesondere von differenzierten Zellen, gefunden, umfassend ein Gehäuse, insbesondere eine zylinderförmige Umhausung aufweisend, mit einer sich von einem ersten Gehäuseende bis zu einem zweiten Gehäuseende erstreckenden longitudinalen Orientierung, eine spiralförmig um eine entlang oder parallel zu der longitudinalen Orientierung ausgerichtete Wickelachse aufgewickelt in dem Gehäuse vorliegende Kunststofffolie mit einem ersten und einem zweiten Folienende und einander gegenüberliegenden Seitenrändern sowie mit einer Innen- und einer gegenüberliegenden Außenseite, wobei auf der Innenseite oder auf der Außenseite oder auf der Innen- und der Außenseite der Kunststofffolie, vorzugsweise auf der Innen- oder der Außenseite der Kunststofffolie, eine Vielzahl an Abstandshaltern vorliegt, eingerichtet und ausgelegt, die Innenseite der gewickelten Kunststofffolie zumindest abschnittsweise, insbesondere vollständig, beanstandet zu halten von der Außenseite von im aufgewickelten Zustand jeweils benachbarten Folienabschnitten, sodass entlang der longitudinalen Orientierung, insbesondere vom ersten Gehäuseende bis zum zweiten Gehäuseende ein flüssiges Medium transferierbar ist.

Als zu kultivierende Zellen kommen im allgemeinen primäre Zellen tierischen oder humanen Ursprungs in Betracht, wobei die erfindungsgemäße Vorrichtung insbesondere geeignet und vorgesehen ist für die Vermehrung von Muskelzellen als primäre Zellen. Mit den zu kultivierenden Zellen sind mit Hilfe der erfindungsgemäßen Vorrichtung kultivierte Lebensmittel zugänglich. Kultivierte Lebensmittel sollen dabei kultiviertes Fleisch ebenso umfassen wie kultivierten Fisch und kultivierte Meeresfrüchte, wobei kultiviertes Fleisch auch kultiviertes Geflügelfleisch umfasst.

Das Gehäuse ist zweckmäßigerweise flüssigkeitsdicht und vorzugsweise auch gasdicht ausgestaltet. Auf diese Weise kann sichergestellt werden, dass während des Betriebs der erfindungsgemäßen Vorrichtung keine Nährflüssigkeit entweicht. Die Dichtigkeit des Gehäuses trägt ebenfalls dazu bei, dass mit der erfindungsgemäßen Vorrichtung die Zellvermehrung wie auch die Ernte der vermehrten Zellen unter axenischen Bedingungen vonstattengehen kann.

Die erfindungsgemäße Vorrichtung kann bevorzugt mit einer Welle ausgestattet sein. Diese Welle ist in dem Gehäuse vorzugsweise entlang oder parallel zu der longitudinalen Orientierung desselben ausgerichtet. Die Welle kann rotierbar wie auch feststehend gestaltet sein. Allerdings hat es sich für viele Anwendungen als vorteilhaft erwiesen, eine rotierbare Welle einzusetzen. Die Welle ist vorzugsweise mit einem Folienende, bevorzugt dem ersten Folienende, der Kunststofffolie verbunden.

Insbesondere für den Fall, dass die an der Kunststofffolie vermehrten Zellen mechanisch geerntet werden sollen, macht man von einem Gehäuse, insbesondere umfassend die zylinderförmige Umhausung, Gebrauch, das einen, insbesondere reversibel verschließbaren, Öffnungsspalt in dem Gehäuse bzw. der Umhausung aufweist mit einem ersten und einem gegenüberliegenden zweiten Öffnungsrand für den Durchtritt der Kunststofffolie, der sich insbesondere entlang der longitudinalen Orientierung erstreckt. Die Freisetzung der an der Oberfläche der Kunststofffolie anhaftenden Zellen gelingt besonders gut, wenn der erste Öffnungsrand oder der zweite Öffnungsrand des Öffnungsspalts als Abschabkante für an der Kunststofffolie anhaftende vermehrte Zellen ausgebildet oder mit einer Abschabkante für die vermehrten Zellen ausgestattet ist.

Die Kunststofffolie liegt in dem Gehäuse spiralförmig aufgewickelt vor. Wird in der erfindungsgemäßen Vorrichtung von einer Welle Gebrauch gemacht, liegt die Kunststofffolie aufgewickelt auf dieser Welle vor. Hierbei kann die Kunststofffolie, insbesondere an ihrem einen Ende, mit der Welle verbunden sein. Auf diese Weise gestaltet sich der Aufwickelvorgang besonders einwandfrei. Bevorzugt ist die Kunststofffolie lösbar mit der Welle verbunden. Auf diese Weise gelingt beim Herausziehen der Kunststofffolie durch einen Öffnungsspalt die komplette Entfernung dieser Folie aus dem Gehäuse. Werden beim Herausziehen der Kunststofffolie aus dem Öffnungsspalt die vermehrten Zellen mechanisch entfernt, kann so eine optimierte Ernte sichergestellt werden.

Die Welle stellt in besonders zweckmäßigen Ausgestaltungen einen Hohlzylinder dar oder liegt in einem Hohlzylinder vor. In letzterem Fall liegt die Kunststofffolie aufgewickelt auf dem Hohlzylinder vor und ist bevorzugt mit diesem, insbesondere an ihrem einen Ende, verbunden. Die Welle bzw. der Hohlzylinder verfügen bevorzugt über einen Durchmesser im Bereich von 5 bis 40 cm, insbesondere im Bereich von 8 bis 25 cm.

Eine besonders komfortable und insbesondere auch zuverlässige Handhabung der erfindungsgemäßen Vorrichtung stellt sich auch dadurch ein, dass diese ferner mit einer Antriebseinheit für die Welle ausgestattet ist. Auf diese Weise kann sowohl der Auf- wie auch der Abwickelvorgang der Kunststofffolie erleichtert werden. Auch wird auf diese Weise ein axenisches Arbeiten auf zulässige Weise sichergestellt. Manuelle Eingriffe können auf diese Weise vermieden werden.

In einer besonders geeigneten Ausführungsform weist die erfindungsgemäße Vorrichtung ferner eine weitere Welle außerhalb des Gehäuses auf. Diese weitere Welle kann dazu genutzt werden, um die anlässlich der mechanischen Gewinnung vermehrter Zellen aus dem Öffnungsspalt heraustretende Kunststofffolie herauszuziehen. Gleichzeitig wird bei diesem Vorgang die Kunststofffolie platzsparend auf der außerhalb des Gehäuses vorliegenden Welle aufgewickelt. Diese Welle kann zum Zweck der Automatisierung mit einer Antriebseinheit verbunden sein.

Das Gehäuse der erfindungsgemäßen Vorrichtung kann ferner mit einer Bodenplatte ausgestattet sein. Auch kann das Gehäuse eine Befestigungsvorrichtung für die in dem Gehäuse vorliegende Welle oder den Hohlzylinder aufweisen. Diese Befestigungsvorrichtung kann in einer Ausführungsform auch an der Bodenplatte angebracht sein. Zwecks Auf- und Abwicklung der Kunststofffolie auf einer im Gehäuse vorliegenden Welle kann eine mit dieser verbundene Antriebseinheit, beispielsweise ein Elektromotor, vorgesehen sein. Für viele Anwendungen hat es sich als zweckmäßig erwiesen, das Gehäuseinnere axenisch auszuführen sowie darauf zu achten, dass die Kunststofffolie unter axenischen Bedingungen in das Gehäuse eingeführt wird.

Für viele Anwendungen hat es sich als besonders vorteilhaft erwiesen, dass sich die Abstandshalter auf der Innen- und/oder der Außenseite, insbesondere auf der Innen- oder der Außenseite, der Kunststofffolie abschnittsweise oder vollständig entlang, insbesondere parallel, der longitudinalen Orientierung erstrecken. Hierbei kann vorgesehen sein, dass sich die Abstandshalter auf der Innen- und/oder, insbesondere oder, der Außenseite der Kunststofffolie vom ersten Gehäuseende oder beanstandet von dem ersten Gehäuseende in Richtung des oder bis zum zweiten Gehäuseende erstrecken, vorzugsweise vom ersten Gehäuseende bis zum gegenüberliegenden zweiten Gehäuseende. Damit die einander benachbarten Folienlagen der gewickelten Kunststofffolie mit einem noch größeren Maß an Sicherheit nicht in Kontakt miteinander stehen bzw. geraten, kann in einer Ausgestaltung vorgesehen sein, dass auf der Innenseite oder auf der Außenseite einander benachbarte Abstandshalter jeweils, insbesondere ununterbrochen, linienförmig gestaltet sind, wobei sich diese linienförmige Ausgestaltung entlang der longitudinalen Orientierung erstreckt.

Die Abstandshalter können in einer besonders geeigneten Ausführungsform in Form einer, insbesondere schlauchförmigen, Luftpolsterfolie vorliegen. Die schlauchförmigen Luftpolster können dabei insbesondere über zwei abschnittsweise alternierend miteinander verbundene Kunststofffolien gebildet sein. Diese schlauchförmigen Luftpolster erstrecken sich vorzugsweise entlang der longitudinalen Orientierung des Gehäuses und liegen insbesondere im Wesentlichen parallel zueinander vor. Alternativ können die Abstandshalter integraler Bestandteil der Kunststofffolie sein. Dies kann beispielsweise dadurch bewerkstelligt werden, dass die Kunststofffolie entsprechend geformte Prägungen aufweist, zum Beispiel erhalten durch thermische und/oder mechanische Manipulation der Kunststofffolie. Um mittels Prägung zu Abstandshaltern zu gelangen, greift man vorzugsweise auf Kunststofffolien aus thermoplastischen Polymeren zurück. Gemäß einer weiteren alternativen Ausgestaltung können die Abstandshalter aus einem Kunststoffmaterial geformt sein, wobei hierfür bevorzugt auf Silikonmassen zurückgegriffen wird. Diese kann auf der Innen- oder auf der Außenseite der Kunststofffolie vorliegen sowie in einigen Fällen auch auf beiden Seiten der Kunststofffolie. Bei den aus Silikonmasse gefertigten Abstandshaltern kann es sich insbesondere auch um eine Vielzahl an tropfenförmigen bzw. im Wesentlichen rundlichen bzw. kreisrunden Erhebungen handeln. Derartig geformte Abstandshalter sind selbstverständlich ebenfalls mittels Prägeformung, wie vorangehend beschrieben, zugänglich.

In einer zweckmäßigen Ausführungsform der erfindungsgemäßen Vorrichtung sind die Abstandshalter benachbarter Umwicklungslagen bzw. von einander benachbarten Folienabschnitten der spiralförmig aufgewickelten Kunststofffolie im Wesentlichen deckungsgleich angeordnet. Auch auf diese Weise gelingt es wirkungsvoll, dass benachbarte Folienlagen stets vollumfänglich hinreichend weit voneinander beabstandet sind und Raum für die Vermehrung der Zellen lassen sowie gleichzeitig den ungehinderten Durchtriff der Nährlösung gewährleisten. Alternativ können die Abstandshalter solcher benachbarten Folienabschnitte auch versetzt zueinander angeordnet sein.

Die Abstandshalter weisen im Allgemeinen eine durchschnittliche Höhe, insbesondere absolute Höhe, im Bereich von 1,0 bis 15,0 mm, bevorzugt im Bereich von 1,5 bis 10,0 mm und besonders bevorzugt im Bereich von 2,0 bis 5,0 mm auf. Der Abstand einander benachbarter Folienbahnabschnitte liegt in der gewickelten Folienbahn in der Regel im Bereich von 0,3 bis 10,0 mm, bevorzugt im Bereich von 1,0 bis 5,0 mm und besonders bevorzugt im Bereich von 1,5 bis 4,0 mm.

Die Kunststofffolie der erfindungsgemäßen Vorrichtung kann für den einmaligen Gebrauch wie auch für den vielfachen Gebrauch vorgesehen sein. Dabei hat sich in beiden Fällen als besonders vorteilhaft herausgestellt, auf solche Ausführungsvarianten zurückzugreifen, bei denen die Kunststofffolie und der Abstandshalter aus sortenreinen Kunststoffen gebildet sind. Dies gestattet es, dieses Produkt einem Recycling zuzuführen, aus dem dann erneut im Wesentlichen identische Produkte zugänglich sind. Zur Mehrfachverwendung der erfindungsgemäßen Vorrichtung bzw. von Komponenten dieser Vorrichtung kann auf etablierte CIP- bzw. CIS-Protokolle und -Methoden ("*Clean-in-Place*" bzw. "*Steam-in-Place*") zurückgegriffen werden. CIP-Protokolle und -Methoden zur Reinigung von Komponenten von Anlagen, wie sie z.B. in der Lebensmittel- und Pharmaindustrie zum Einsatz kommen, sind dem Fachmann, hinlänglich bekannt. Zwecks Reinigung gemäß einem CIP/CIS-Protokoll ist der Herstell- bzw. Verarbeitungsprozess in der Regel zu unterbrechen, und die Anlage ist von allen Prozessmaterialien zu befreien.

Die, insbesondere beschichtete oder behandelte, Kunststofffolie der erfindungsgemäßen Vorrichtung stellt eine Wachstumsoberfläche dar. Damit auf der Kunststofffolie Zellen anhaften bzw. anwachsen und sich anschließend in Gegenwart einer Nährlösung vermehren können, hat es sich als vorteilhaft erwiesen, die Innenseite, die Außenseite oder die Innen- und die Außenseite der Kunststofffolie in einer Weise zu behandeln, dass den Zellen geeignete Anlagerungsmöglichkeiten geboten werden. Vielfach gelingt dies besonders gut, indem man einer Oberfläche jedenfalls eine gewisse Polarität verleiht. Demgemäß kann für die Kunststofffolie auf solche Innenseiten und/oder Außenseiten zurückgegriffen werden, die plasmabehandelt, coronabehandelt oder laugenbehandelt worden sind.

Auch hat es sich für viele Anwendungen als hinreichend erwiesen, die Innenseite, die Außenseite oder die Innen- und die Außenseite der Kunststofffolie mit einer Oberflächenrauigkeit auszustatten. Hierbei sind solche Innen- bzw. Außenseiten bevorzugt, die über eine durchschnittliche Oberflächenrauigkeit Sₐ im Bereich von 0,01bis 100 µ, bevorzugt im Bereich von 0,1 bis 75 µm und besonders bevorzugt im Bereich von 1,0 bis 50 µm, jeweils ermittelt gemäß DIN EN ISO 25178 (2010-2020), verfügen.

Des Weiteren kann eine für das Anhaften bzw. Anwachsen und Vermehren von Zellen besonders vorteilhafte Unterlage auch dadurch erhalten werden, dass man die Innenseite, die Außenseite oder die Innen- und die Außenseite der Kunststofffolie mit einer geeigneten Beschichtung versieht. Hierbei sind solche Beschichtungen bevorzugt auf Basis von oder gebildet aus Gelatine, Poly-L-Lysin, Poly-D-Lysin, Poly-Ornithine, Collagen, insbesondere Collagen I, II oder IV, Fibronektin, Laminin, Elastin, Entactin, Vitronektin, Osteopontin, Matrigel, Hydrogel, Aginatgel, Lactatgel und/oder Bestandteilen der Basalmembran aus Engelbreth-Holm-Swarm (EHS)-Mäusetumoren.

Als ganz besonders bevorzugt haben sich, insbesondere vollflächige, Beschichtungen basierend auf thermoresponsivem Material für die Innenseite, die Außenseite oder die Innen- und die Außenseite der Kunststofffolie erwiesen. Demgemäß kann vorgesehen sein, dass die Oberfläche der Innenseite, der Außenseite oder der Innen- und der Außenseite der Kunststofffolie zumindest abschnittsweise, insbesondere im Wesentlichen vollständig, bevorzugt vollständig, mit einem thermoresponsiven Material ausgestattet ist oder dass die Kunststofffolie ein thermoresponsives Material umfasst oder hieraus besteht.

Die Kunststofffolie kann aus einer oder mehreren Lagen gebildet sein. In einer Ausführungsform kann es sich bei der Kunststofffolie um eine Coextrusionsfolie handeln, enthaltend beispielsweise 3, 5 oder 7 Einzellagen.

Mit der erfindungsgemäßen Vorrichtung können vielfältige Anwendungen realisiert werden. So kann die erfindungsgemäße Vorrichtung einerseits angepasst werden für Anwendungen im Labormaßstab und andererseits für die Produktion von kultivierten Lebensmitteln wie kultiviertem Fleisch im industriellen Maßstab. Die Kunststofffolie kann demgemäß über eine Länge im Bereich von 2 bis 1000 m verfügen. Alternativ sowie insbesondere zusätzliche kann dabei vorgesehen sein, dass die Breite der Kunststofffolie im Bereich von 0,25 bis 2,0 m liegt. Für viele Anwendungen hat es sich als bevorzugt erwiesen, die Dicke der Kunststofffolie derart einzustellen, dass sie im Bereich von 0,05 bis 1,0 mm, bevorzugt im Bereich von 0,05 bis 0,5 mm und besonders bevorzugt im Bereich von 0,1 bis 0,3 mm liegt.

Die der Erfindung zugrunde liegende Aufgabe wird insbesondere auch dadurch besonders zuverlässig und erfolgreich gelöst, dass in bevorzugten Ausführungsformen die Kunststofffolie eine Biegefestigkeit größer oder gleich 1000 mN^{∗}cm², bevorzugt größer oder gleich 2000 mN^{∗}cm², z.B. im Bereich von 1000 bis 10000 mN^{∗}cm² oder 2000 bis 7500 mN^{∗}cm², jeweils bestimmt gemäß DIN 53362:2003-10, aufweist. Auf diese Weise kann insbesondere auch sichergestellt werden, dass selbst bei geringen Spaltbreiten zwischen benachbarten Lagen der aufgewickelten Kunststofffolie diese auch bei längeren Einsatzzeiten der erfindungsgemäßen Vorrichtung vermittels der Abstandshalter nicht in Kontakt miteinander treten.

Mit der erfindungsgemäßen Vorrichtung ist es möglich, dass das Gehäuse, die Kunststofffolie oder die Abstandshalter, bevorzugt das Gehäuse, die Kunststofffolie und die Abstandshalter, auf Polymeren aus nachwachsenden Rohstoffen basieren. Ferner kann vorgesehen sein, dass für das Gehäuse, die Kunststofffolie oder die Abstandshalter auf Polymilchsäure, Polyolefinen, insbesondere Polyethylen oder Polypropylen, Polyhydroxyalkanoaten, Polycaprolactonen, Polyestern, Polyamiden oder Stärke basieren, wobei bevorzugt auf Polymilchsäure zurückgegriffen wird.

Die erfindungsgemäße Vorrichtung kann in besonders geeigneten Ausführungsformen mindestens eine Einlassöffnung, insbesondere im Bereich des ersten Gehäuseendes, und mindestens eine Auslassöffnung, insbesondere im Bereich des zweiten Gehäuseendes aufweisen. Alternativ sowie insbesondere zusätzlich kann die erfindungsgemäße Vorrichtung ferner mindestens eine Zuführleitung für die Nährlösung, insbesondere im Bereich des ersten Gehäuseendes, und mindestens eine Abflussleitung für die Nährlösung, insbesondere im Bereich des zweiten Gehäuseendes, aufweisen. Sie Seitenränder der Kunststofffolie sind zweckmäßigerweise benachbart zum ersten bzw. zweiten Gehäuseende angeordnet. Auf diese Weise kann das Volumen des Gehäuses bestmöglich ausgenutzt werden, um einen hohen Ernteertrag zu erzielen.

Eine besonders zuverlässige Zellvermehrung lässt sich insbesondere auch mit solchen erfindungsgemäßen Vorrichtungen erhalten, die mindestens einen Temperatur-, Sauerstoff-, Kohlendioxid-, pH-, Laktat-, Fructose-, Glukose- und/oder, insbesondere und, Leitfähigkeitssensor, vorzugsweise mindestens einen Sauerstoff-, Laktat- und Glukosesensor, insbesondere im Bereich der oder in der Zuführleitung und/oder im Bereich der oder in der Abflussleitung aufweisen. Alternativ sowie insbesondere zusätzlich kann vorgesehen sein, dass mindestens ein Sensor vorgesehen ist, eingerichtet und ausgelegt für die Bestimmung der Durchflussrate der Nährlösung in der Zuführleitung oder benachbart zur Zuführleitung und/ oder, insbesondere und, mindestens einen Sensor eingerichtet und ausgelegt für die Bestimmung der Durchflussrate der Nährlösung in der Abflussleitung oder benachbart zur Abflussleitung.

Um sterile sowie insbesondere axenische Arbeitsbedingungen im Gehäuse aufrecht zu erhalten, hat es sich für viele Anwendungen als zweckmäßig erwiesen, mindestens einen Sterilfilter in den Zu- oder Abführleitungen, vorzugsweise in den Zu- und Abführleitungen, vorzusehen bzw. zu integrieren, der für den Transfer von Gasen vorgesehen ist. Darüber hinaus ist es in diesem Zusammenhang von Vorteil, alternativ sowie insbesondere zusätzlich in den Zu- oder Abführleitungen, vorzugsweise in den Zu- und Abführleitungen, für den Transfer flüssiger wie gasförmiger Substanzen einen Sterilkonnektor vorzusehen bzw. zu integrieren.

In einer sehr zweckmäßigen Weiterentwicklung umfasst die erfindungsgemäße Vorrichtung ebenfalls einen Auffangbehälter verbunden oder verbindbar mit der Abführleitung für die Aufnahme von aus dem Gehäuse abfließende Nährlösung. Dieser Auffangbehälter kann dabei auch eine Rühreinheit und/oder eine Temperiereinheit umfassen. Des Weiteren hat es sich als vorteilhaft erwiesen, wenn dieser Behälter mit einer Begasungseinheit in Wirkverbindung steht oder bringbar ist. Diese Begasungseinheit bzw. deren Zuführleitung zum Auffangbehälter kann ebenfalls mit mindestens einem Sterilfilter ausgestattet sein. Dieser Behälter kann demgemäß als Konditioniergefäß für die Nährlösung dienen. Darüber hinaus kann eine optimierte Arbeitsweise insbesondere auch dadurch gewährleistet werden, dass der Auffangbehälter mit einem Temperatur-, Sauerstoff-, Kohlendioxid-, pH-, Lktat-, Fructose- und/oder, insbesondere und, Leitfähigkeitssensor ausgestattet ist. Mithilfe des geschilderten Auffangbehälters kann die über die Abflussleitung entnommene Nährlösung aufbereitet und dem Gehäuse wieder zugeführt werden.

Ist die Kultivierung der Zellen abgeschlossen, können die an der Kunststofffolie anhaftenden vermehrten Zellen auf vielfältige Weise hiervon abgelöst und gewonnen werden. Beispielsweise kann gemäß einer Ausführungsform die Kunststofffolie durch einen in dem Gehäuse vorliegenden Öffnungsspalt herausgezogen werden, wobei die vermehrten Zellen entlang einer Kante oder Wulst mechanisch freigesetzt/ abgeschabt werden. Zwecks Erleichterung der Gewinnung der freigesetzten/abgeschabten Zellen hat es sich als zweckmäßig erwiesen, den Öffnungsspalt bzw. den oder die Ränder des Öffnungsspalts, vorzugsweise über eine im Gehäuse angeordnete Düse, mit einem flüssigen oder gasförmigen Medium zu beaufschlagen. Die in dem Gehäuse aufgewickelt vorliegende Kunststofffolie wird bei diesem Vorgang abgewickelt. Neben der mechanischen Gewinnung der vermehrten Zellen hat sich als besonders vorteilhaft erwiesen, diese vermehrten Zellen auf physikalischem Wege, beispielsweise durch Temperaturänderung, insbesondere unter Einbindung von thermoresponsivem Beschichtungsmaterial, Ultraschallbehandlung oder Lichteinwirkung, oder auf chemischem Wege, beispielsweise durch Behandlung mit Trypsin, freizusetzen. Die physikalische und die chemische Freisetzung findet in zweckmäßigen Ausführungsformen im Gehäuse der erfindungsgemäßen Vorrichtung statt. Die freigesetzten Zellen können sodann zusammen mit der Nährlösung aus dem Gehäuse abgeführt und isoliert werden.

Das Gefäß für die Nährlösung, auch Konditionierungsgefäß genannt, kann insbesondere auch inklusive sämtlicher Zuführ- und Abflussleitungen in dem Gehäuse eingesetzt vorliegen.

Das Konditionierungsgefäß ist vorzugsweise derart gestaltet, dass es auch mit handelsüblichen Prozessleitsystemen betrieben werden kann.

Mit der vorliegenden Erfindung geht die überraschende Erkenntnis einher, dass sich mit der erfindungsgemäßen Vorrichtung entlang des Transportweges der Nährstofflösung ein bevorzugter Konzentrationsgradient eingestellt bzw. beibehalten werden kann. Mit der erfindungsgemäßen Vorrichtung wird folglich ein störungsfreier Durchtriff der Nährlösung gewährleistet. Mit der erfindungsgemäßen Vorrichtung wird zudem sichergestellt, dass die Abstände zwischen benachbarten Folienabschnitten hinreichend groß sind für den Durchtritt der Nährlösung, und zwar auch nach fortgeschrittener Vermehrung der an der Kunststofffolie anhaftenden Zellen. Mit der erfindungsgemäßen Vorrichtung kann ein Durchfluss, insbesondere ein laminarer Durchfluss, der Nährlösung sichergestellt und aufrechterhalten werden. Es gelingt zuverlässig eine scherarme Versorgung mit Nährstoffen. Im Ergebnis wird mit der erfindungsgemäßen Vorrichtung ein Kultivierungsgefäß zur Expansion von adhärenten Zellen zur Verfügung gestellt, mit dem kultivierte Lebensmittel, insbesondere kultiviertes Fleisch, kultivierter Fisch oder kultivierte Meeresfrüchte, im industriellen Maßstab zuverlässig und in gleichbleibend hoher Qualität verfügbar gemacht werden können. Von besonderem Vorteil bei der erfindungsgemäßen Vorrichtung ist auch, dass das Gehäuse nach erfolgter Benutzung wiederverwendet werden kann. Mit der Kunststofffolie als Wachstumsoberfläche als Bestandteil der erfindungsgemäßen Vorrichtung lassen sich die Kosten für die Vermehrung bzw. Kultivierung von Zellen, insbesondere differenzierten Zellen, gegenüber gattungsgemäßen Verfahren signifikant verringern.

Weitere Merkmale und Vorteile der Erfindung ergeben sich aus der nachgehenden Beschreibung, in der bevorzugte Ausführungsformen der Erfindung beispielhaft anhand schematischer Zeichnungen erläutert sind. Dabei zeigen:
- Figur 1: eine schematische perspektivische Darstellung der erfindungsgemäßen Vorrichtung;
- Figur 2: eine schematische Querschnittsansicht durch die erfindungsgemäße Vorrichtung gemäß Figur 1;
- Figur 3: eine schematische Darstellung eines Ausschnitts der erfindungsgemäßen Vorrichtung gemäß Figur 1;
- Figur 4: eine schematische Darstellung einer weiteren Ausführungsform der erfindungsgemäßen Vorrichtung;
- Figur 5: eine schematische Darstellung einer weiteren Ausführungsform der erfindungsgemäßen Vorrichtung;
- Figur 6: eine schematische Darstellung eines Ausschnitts der erfindungsgemäßen Vorrichtung gemäß Figur 5; und
- Figur 7: eine schematische Darstellung einer weiteren Ausführungsform der erfindungsgemäßen Vorrichtung.

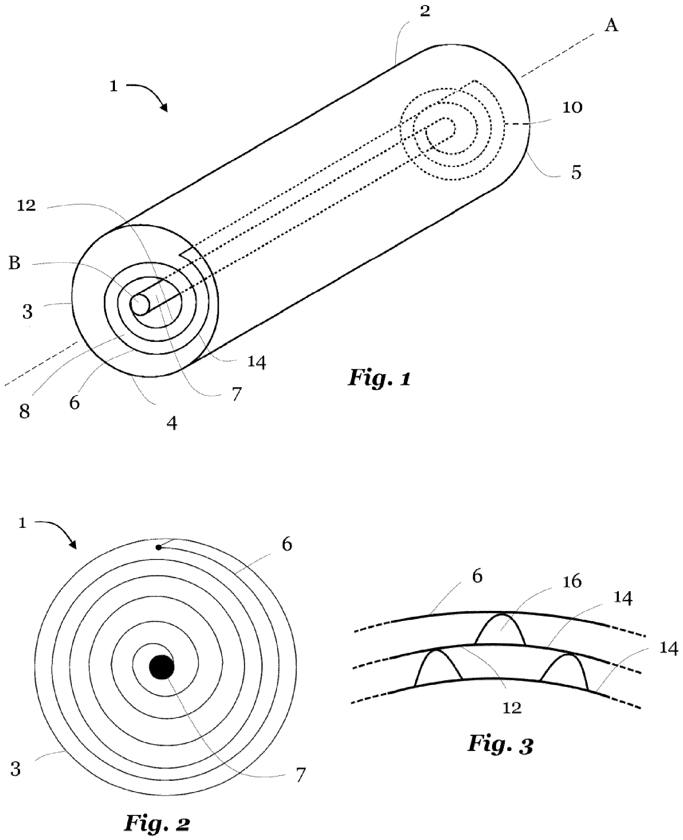

Figur 1 gibt eine schematische perspektivische Darstellung einer erfindungsgemäßen Vorrichtung (1) zur Vermehrung von Zellen, z.B. differenzierten Zellen, wieder. Zur übersichtlicheren Darstellung sind darin die Abstandshalter nicht gezeigt. Diese Vorrichtung (1) umfasst in der dargestellten Ausführungsform ein Gehäuse (2) mit einer zylinderförmigen Umhausung (3). Das Gehäuse (2) weist eine sich von einem ersten Gehäuseende (4) bis zu einem zweiten Gehäuseende (5) erstreckende longitudinale Orientierung (A) auf. Kolinear zu dieser longitudinalen Orientierung (A) ausgerichtet liegt eine Wickelachse (B) vor, die im vorliegenden Fall als Welle (7) verkörpert ist. Um dieser Welle (7) ist eine spiralförmig aufgewickelte Kunststofffolie (6) angeordnet. Diese Kunststofffolie (6) liegt demgemäß in dem Gehäuse (2) vor. Sie ist mit einem ersten und einem zweiten Folienende (8, 10) und einander gegenüberliegenden Seitenrändern sowie mit einer Innen- und einer gegenüberliegenden Außenseite (12, 14) ausgestattet. Figur 2 zeigt eine schematische Querschnittsansicht durch eine Vorrichtung (1) gemäß Figur 1. Wie dem in Figur 3 wiedergegebenen Ausschnitt der Vorrichtung gemäß Figur 1 zu entnehmen ist, sind auf der Außenseite (14) der Kunststofffolie (6) eine Vielzahl an Abstandshaltern (16) präsent. Diese verfügen über eine longitudinale Orientierung in der dargestellten Ausführungsform. Diese Abstandshalter (16) halten in der aufgewickelten Kunststofffolie (6) die Innenseite (12) beanstandet von der Außenseite (14) des im aufgewickelten Zustand benachbarten Folienabschnitts. Auf diese Weise kann entlang der longitudinalen Orientierung vom ersten Gehäuseende (4) bis zum zweiten Gehäuseende (5) Flüssigkeit transferiert werden. Bevorzugt halten die Abstandshalter (16) in der aufgewickelten Kunststofffolie (6) die Innenseite (12) über alle jeweils einander benachbarten Umwicklungslagen beanstandet von der Außenseite (14).

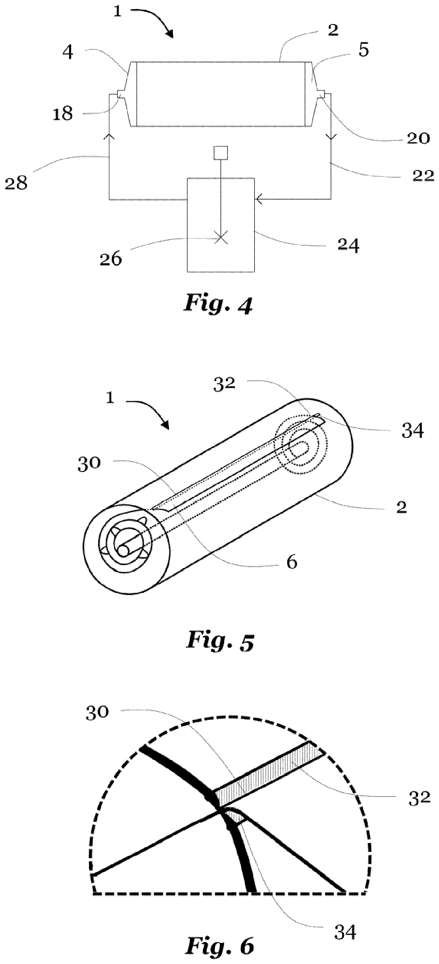

Figur 4 zeigt eine weitere, sehr zweckmäßige Ausführungsform einer erfindungsgemäßen Vorrichtung (1), bei der eine Einlassöffnung (18) im Bereich des ersten Gehäuseendes (4) und eine Auslassöffnung (20) im Bereich des zweiten Gehäuseendes (5) vorliegt. An der Auslassöffnung (20) ist eine Abflussleitung (22) angeordnet, über die Nährlösung aus dem Gehäuse (2) abfließen und in einen Auffangbehälter (24) aufgenommen und gesammelt werden kann, z.B. um darin temperiert und um frische Nährlösung ergänzt zu werden. Dieser Auffangbehälter (16) ist in der dargestellten Variante mit einer Rühreinheit (26) ausgestattet. Die aufbereitete Nährlösung kann sodann über die Zuführleitung (28) zur im Bereich des ersten Gehäuseendes (4) angeordneten Einlassöffnung (18) transferiert und wieder in das Gehäuse (2) der Vorrichtung eingebracht werden.

Figur 5 zeigt eine weitere Ausführungsform der erfindungsgemäßen Vorrichtung (1). Bei dieser Ausführungsform ist die zylinderförmigen Umhausung des Gehäuses (2) mit einem reversibel verschließbaren Öffnungsspalt (30) mit einem ersten und einem gegenüberliegenden zweiten Öffnungsrand (32, 34) ausgestattet. Durch diesen Öffnungsspalt (30), der sich in der dargestellten Ausführungsform entlang der longitudinalen Orientierung (A) erstreckt, kann die Kunststofffolie (6) hindurchtreten.

Figur 6 entnimmt man einen Ausschnitt des Öffnungsspalts (30) mit dem ersten und dem zweiten Öffnungsrand (32, 34), wie in der Vorrichtung gemäß Figur 5 einsetzbar. In der dargestellten Ausführungsform sind der erste Öffnungsrand und der zweite Öffnungsrand des Öffnungsspalts als Abschabkanten für an der Kunststofffolie anhaftende vermehrte Zellen ausgebildet.

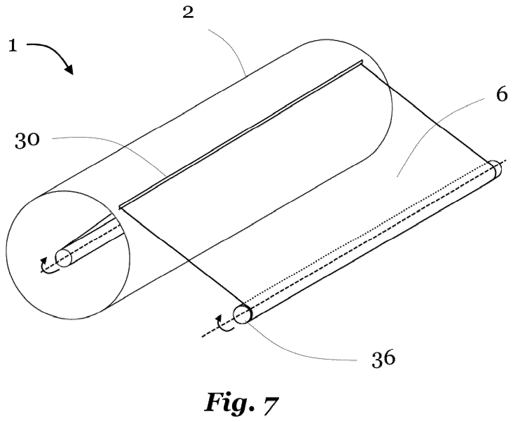

Figur 7 zeigt eine schematische Darstellung einer weiteren Ausführungsform der erfindungsgemäßen Vorrichtung (1). Diese ist mit einer weiteren Welle (36) ausgestattet, die außerhalb des Gehäuses (2) vorliegt und mit deren Hilfe das Abziehen und Aufwickeln der aus dem Öffnungsspalt (30) heraustretenden Kunststofffolie (6) automatisiert werden kann.

Die in der vorstehenden Beschreibung, in den Ansprüchen und in den Zeichnungen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Vorrichtung zur Vermehrung von Zellen, insbesondere von differenzierten Zellen, umfassend
ein Gehäuse, insbesondere mit einer zylinderförmiger Umhausung, mit einer sich von einem ersten Gehäuseende bis zu einem zweiten Gehäuseende erstreckenden longitudinalen Orientierung,
eine spiralförmig um eine entlang oder parallel zu der longitudinalen Orientierung ausgerichtete Wickelachse aufgewickelt in dem Gehäuse vorliegende Kunststofffolie mit einem ersten und einem zweiten Folienende und gegenüberliegenden Seitenrändern sowie mit einer Innen- und einer gegenüberliegenden Außenseite, wobei auf der Innenseite oder auf der Außenseite oder auf der Innen- und der Außenseite der Kunststofffolie, vorzugsweise auf der Innen- oder der Außenseite der Kunststofffolie, eine Vielzahl an Abstandshaltern vorliegt, eingerichtet und ausgelegt, die Innenseite der gewickelten Kunststofffolie abschnittsweise oder im Wesentlichen vollständig, insbesondere vollständig, im aufgewickelten Zustand beanstandet zu halten von der Außenseite, sodass entlang der longitudinalen Orientierung, insbesondere vom ersten Gehäuseende bis zum zweiten Gehäuseende ein flüssiges Medium transferierbar ist.

2. Vorrichtung nach Anspruch 1, ferner umfassend
eine, insbesondere rotierbare, Welle ausgerichtet entlang oder parallel zu der longitudinalen Orientierung, wobei die Kunststofffolie spiralförmig aufgewickelt auf der Welle vorliegt, wobei vorzugsweise die Kunststofffolie mit der Welle verbunden ist.

3. Vorrichtung nach Anspruch 2, ferner umfassend
eine Antriebseinheit für die Welle.

4. Vorrichtung nach einem der vorangehenden Ansprüche, **dadurch gekennzeichnet, dass**
das Gehäuse, insbesondere die zylinderförmige Umhausung, einen, insbesondere reversibel verschließbaren, Öffnungsspalt mit einem ersten und einem gegenüberliegenden zweiten Öffnungsrand für den Durchtritt der Kunststofffolie aufweist, der sich insbesondere entlang der longitudinalen Orientierung erstreckt.

5. Vorrichtung nach einem der vorangehenden Ansprüche, ferner umfassend mindestens eine Einlassöffnung, insbesondere im Bereich des ersten Gehäuseendes, und mindestens eine Auslassöffnung, insbesondere im Bereich des zweiten Gehäuseendes.

6. Vorrichtung nach einem der vorangehenden Ansprüche, ferner umfassend mindestens eine Zuführleitung für eine Nährlösung, insbesondere im Bereich des ersten Gehäuseendes, und mindestens eine Abflussleitung für die Nährlösung, insbesondere im Bereich des zweiten Gehäuseendes.

7. Vorrichtung nach einem der vorangehenden Ansprüche, ferner umfassend mindestens einen Temperatur-, Sauerstoff-, pH-, Lactat-, Fructose-, Glukose- und/oder, insbesondere und, Leitfähigkeitssensor, vorzugsweise mindestens einen Sauerstoff-, Kohlenstoffdioxyd-, Laktat- und Glukosesensor, insbesondere im Bereich der oder in der Zuführleitung und/ oder im Bereich der oder in der Abflussleitung, und/oder, insbesondere und, mindestens einen Sensor eingerichtet und ausgelegt für die Bestimmung der Durchflussrate der Nährlösung in der Zuführleitung oder benachbart zur Zuführleitung und/oder, insbesondere und, mindestens einen Sensor eingerichtet und ausgelegt für die Bestimmung der Durchflussrate der Nährlösung in der Abflussleitung oder benachbart zur Abflussleitung.

8. Vorrichtung nach einem der vorangehenden Ansprüche, ferner umfassend eine weitere Welle, außerhalb des Gehäuses vorliegend, ausgelegt und eingerichtet zum Abziehen und Aufwickeln der aus dem Öffnungsspalt heraustretenden Kunststofffolie.

9. Vorrichtung nach einem der vorangehenden Ansprüche, **dadurch gekennzeichnet, dass**
die Innenseite oder die Außenseite oder die Innen- und die Außenseite der Kunststofffolie plasmabehandelt, coronabehandelt oder laugenbehandelt ist oder eine Beschichtung auf Basis von oder gebildet aus Gelatine, Poly-L-Lysin, Poly-D-Lysin, Poly-Ornithine, Collagen, insbesondere Collagen I, II oder IV, Fibronektin, Laminin, Elastin, Entactin, Vitronektin, Osteopontin, Matrigel, Hydrogel, Aginatgel, Lactatgel und/oder Bestandteilen der Basalmembran aus Engelbreth-Holm-Swarm (EHS)-Mäusetumoren aufweist.

10. Vorrichtung nach einem der vorangehenden Ansprüche, **dadurch gekennzeichnet, dass**
die Innenseite oder die Außenseite oder die Innen- und die Außenseite der Kunststofffolie eine Oberflächenrauigkeit aufweisen, insbesondere eine durchschnittliche Oberflächenrauigkeit Sₐ im Bereich von 0,01 bis 100 µm, bevorzugt im Bereich von 0,1 bis 75 µm und besonders bevorzugt im Bereich von 1,0 bis 50 µm, jeweils ermittelt gemäß DIN EN ISO 25178 (2010-2020).

11. Vorrichtung nach einem der vorangehenden Ansprüche, **dadurch gekennzeichnet, dass**
die Kunststofffolie eine Biegefestigkeit größer oder gleich 1000 mN^{∗}cm², bevorzugt größer oder gleich 2000 mN^{∗}cm², besonders bevorzugt im Bereich von 1000 bis 10000 mN^{∗}cm² und insbesondere im Bereich von 2000 bis 7500 mN^{∗}cm² , jeweils bestimmt gemäß DIN 53362:2003-10, aufweist.

12. Vorrichtung nach einem der vorangehenden Ansprüche, **dadurch gekennzeichnet, dass**
die Innenseite oder die Außenseite oder die Innen- und die Außenseite der Kunststofffolie zumindest abschnittsweise, insbesondere im Wesentlichen vollflächig, mit einer thermoresponsiven Oberfläche ausgestattet sind oder dass die Kunststofffolie ein thermoresponsives Material umfasst oder hieraus besteht und/oder, insbesondere und,
dass sich die Abstandshalter auf der Innen- und/ oder der Außenseite der Kunststofffolie abschnittsweise oder vollständig entlang, insbesondere parallel, der longitudinalen Orientierung erstrecken und/oder, insbesondere und,
dass sich die Abstandshalter auf der Innen- und/oder der Außenseite der Kunststofffolie vom ersten Gehäuseende oder beanstandet von dem ersten Gehäuseende in Richtung des oder bis zum zweiten Gehäuseende erstrecken.

13. Vorrichtung nach einem der vorangehenden Ansprüche, **dadurch gekennzeichnet, dass**
die Abstandshalter in Form einer, insbesondere schlauchförmigen, Luftpolsterfolie vorliegen, wobei die schlauchförmigen Luftpolster insbesondere gebildet sind durch zwei abschnittsweise alternierend miteinander verbundene Kunststofffolien und/oder, insbesondere oder,
dass die Abstandshalter integraler Bestandteil der Kunststofffolie sind, insbesondere als Folienprägung ausgebildet sind, und/oder, insbesondere oder, dass die Abstandshalter in Form eines Kunststoffmaterials, insbesondere einer Silikonmasse, auf der Innenseite, die Außenseite oder die Innen- und die Außenseite der Kunststofffolie vorliegen.

14. Vorrichtung nach einem der vorangehenden Ansprüche, **dadurch gekennzeichnet, dass**
die Abstandshalter benachbarter Umwicklungslagen im Wesentlichen deckungsgleich angeordnet sind.

15. Vorrichtung nach einem der vorangehenden Ansprüche, **dadurch gekennzeichnet, dass**
auf der Innenseite oder auf der Außenseite einander benachbarte Abstandshalter jeweils, insbesondere ununterbrochen, linienförmig gestaltet sind, wobei sich diese linienförmige Ausgestaltung entlang der longitudinalen Orientierung erstreckt.

16. Vorrichtung nach einem der Ansprüche 4 bis 15, **dadurch gekennzeichnet, dass** der erste Öffnungsrand oder der zweite Öffnungsrand des Öffnungsspalts als Abschabkante für an der Kunststofffolie anhaftende vermehrte Zellen ausgebildet oder mit einer Abschabkante für die vermehrten Zellen ausgestattet ist.

17. Vorrichtung nach einem der Ansprüche 6 bis 16, **dadurch gekennzeichnet, dass** in den Zu- oder Abführleitungen, vorzugsweise in den Zu- und Abführleitungen, die für den Transfer von Gasen vorgesehen sind, mindestens ein Sterilfilter vorgesehen oder integriert ist und/oder, insbesondere und, dass in die Zu- oder Abführleitungen, vorzugsweise in den Zu- und Abführleitungen, für den Transfer flüssiger oder gasförmiger Substanzen mindestens ein Sterilkonnektor vorgesehen oder integriert ist.

18. Vorrichtung nach einem der vorangehenden Ansprüche, ferner umfassend einen Auffangbehälter verbunden oder verbindbar mit der Abflussleitung für die Aufnahme von aus dem Gehäuse abfließende Nährlösung.

19. Vorrichtung nach Anspruch 18, **dadurch gekennzeichnet, dass** der Auffangbehälter eine Rühreinheit und/oder eine Temperiereinheit umfasst und gegebenenfalls mit einer Begasungseinheit in Wirkverbindung steht oder bringbar ist.

20. Vorrichtung nach Anspruch 19, ferner umfassend
eine Verbindung zu der Zuführleitung.

21. Vorrichtung nach einem der Ansprüche 18 bis 20, ferner umfassend einen Temperatur-, Sauerstoff-, Kohlenstoffdioxyd-, pH-, Laktat-, Glukose-, Fruktose- und/oder, insbesondere und, Leitfähigkeitssensor.

22. Verwendung der Vorrichtung gemäß einem der vorangehenden Ansprüche für die Herstellung von kultivierten Lebensmitteln, insbesondere von kultiviertem Fleisch, kultiviertem Fisch oder kultivierten Meeresfrüchten, oder für die Gewinnung von vermehrten differenzierten oder undifferenzierten Zellen, insbesondere von vermehrten differenzierten Zellen.
